**Europäisches Patentamt**

**European Patent Office**

**Office européen des brevets**

(19)

(11) Numéro de publication : **0 467 742 A1**

(12) # DEMANDE DE BREVET EUROPEEN

(21) Numéro de dépôt : **91401866.8**

(22) Date de dépôt : **05.07.91**

(51) Int. Cl.⁵ : **C07C 205/12,** C07C 201/12, C07B 39/00

(30) Priorité : **16.07.90 FR 9009023**
**16.07.90 FR 9009025**

(43) Date de publication de la demande :
**22.01.92 Bulletin 92/04**

(84) Etats contractants désignés :
**AT BE CH DE DK ES FR GB GR IT LI LU NL SE**

(71) Demandeur : **RHONE-POULENC CHIMIE**
**25, quai Paul Doumer**
**F-92408 Courbevoie Cédex (FR)**

(72) Inventeur : **Forat, Gérard**
**276, rue Duguesclin**
**F-69003 Lyon (FR)**
Inventeur : **Gilbert, Laurent**
**72, rue Vauban**
**F-69006 Lyon (FR)**
Inventeur : **Langlois, Bernard**
**91, rue Duguesclin**
**F-69006 Lyon (FR)**

(74) Mandataire : **Ricalens, François et al**
**RHONE-POULENC CHIMIE Service Brevets**
**Chimie 25, quai Paul Doumer**
**F-92408 Courbevoie (FR)**

(54) **Procédé de synthèse de dérivés fluorés.**

(57)    La présente invention a pour objet un procédé de synthèse de composés organo fluorés.
Le procédé se fait par échange avec des fluorures lequel est réalisé sous l'action d'ultra son.
Application à la synthèse organique.

EP 0 467 742 A1

La présente invention a pour objet la synthèse de dérivés fluorés. Elle a plus particulièrement pour objet la synthèse de dérivés fluorés à partir de dérivés halogénés tels que les dérivés chlorés.

Les composés fluorés sont en général difficiles d'accès. La réactivité du fluor est telle qu'il n'est pas possible d'obtenir directement les dérivés fluorés.

Une des techniques les plus employées pour fabriquer le dérivé fluoré consiste à faire réagir un dérivé halogéné parfois nitré, en général chloré, pour échanger l'halogène avec un fluor minéral, en général un fluorure de métal alcalin, en général de poids atomique élevé.

En général, le fluorure utilisé est le fluorure de potassium qui constitue un compromis économique satisfaisant.

Dans ces conditions, de nombreux procédés tels que par exemple ceux décrits dans le certificat d'addition N° 2 353 516 et dans l'article Chem. Ind. (1978) - 56 ont été décrits et mis en oeuvre industriellement pour obtenir des fluorures d'aryle, aryles sur lesquels sont greffés des groupements électro-attracteurs.

Sauf dans les cas où le substrat est particulièrement adapté à ce type de synthèse, cette technique présente des inconvénients dont les principaux sont ceux que l'on va analyser ci-après.

La réaction est lente et nécessite, en raison d'un temps de séjour élevé, des investissements importants.

Encore faut-il mentionner que pour être utilisable industriellement, cette technique doit être utilisée à des températures élevées, en général aux alentours de 250°C, c'est-à-dire dans la zone où les solvants orga niques les plus stables commencent à se décomposer.

Enfin, les rendements restent médiocres, à moins que l'on utilise des réactifs particulièrement chers comme les fluorures de métal alcalin dont la masse atomique est supérieure à celle du potassium.

Compte-tenu du prix de ces métaux alcalins, leur utilisation industrielle n'est justifiable que pour des produits à haute valeur ajoutée et lorsque l'amélioration de rendement et des cinétiques le justifie, ce qui est rarement le cas.

C'est pourquoi un des buts de la présente invention est de fournir une technique qui soit susceptible de faciliter l'échange entre les halogènes tels que le chlore et le fluor en améliorant significativement le rendement d'échange.

Un autre but de la présente invention est de fournir une technique qui améliore suffisamment la cinétique d'échange pour que l'on puisse envisager l'utilisation de température significativement plus basse que celle employée auparavant ; cette abaissement de température devra être suffisant pour permettre l'utilisation de réactifs qui jusqu'à présent n'étaient pas utilisés en raison de leur instabilité à la température à laquelle s'effectuait l'échange.

Un autre but de la présente invention est de fournir un procédé d'échange entre le fluor et les halogénes de nombre atomique plus élevé utilisant cette technique.

Ces buts et d'autres qui apparaîtront par la suite sont atteints au moyen d'un procédé de synthèse de composés organo fluorés par échange avec des fluorures minéraux, où l'échange est réalisé sous l'action d'ultra-sons. De manière surprenante, il a été montré que la réaction d'échange entre l'ion fluorure sous forme d'un alcalin ou d'un alcalino-terreux avec un ion halogéné de rang plus élevé (c'est-à-dire de nombre atomique plus élevé) lié avec un carbone, que ce dernier soit aromatique ou aliphatique, était significativement amélioré par l'utilisation d'ultra-sons.

Ceci est particulièrement surprenant, d'une part parce-que des essais avaient été réalisés auparavant et qu'ils s'étaient révélés négatifs (cf.Journal of Fluorine Chemistry, 46, (1990) 529-537"HALEX" FLUORINATION OF CHLORINATED BENZALDEHYDES AND BENZOYL CHLORIDES) ; d'autre part, parce que les ultra-sons (cf. Sonochemistry - The Use of ultrasonic waves in Synthetic Organic chemistry, Cathy EINHORN, Jacques EINHORN, Jean-Louis LUCHE, in Synthesis, [Journal of Synthetic Organic Chemistry] P.787) sont, en général utilisés pour des réactions intervenant à basse température et en milieu dilué, alors que dans l'utilisation visée par la présente demande, ils sont utilisés à température relativement élevée, c'est-à-dire à une température significativement au-dessus de la température ambiante en général à une température d'au moins 100° C (sauf lorsque cela est précisé autrement les zéros ne sont pas des chiffres significatifs).

Il n'est pas possible de déterminer de façon précise la raison pour laquelle l'essai mentionné dans la littérature n'a donné aucun résultat. Toutefois, l'utilisation des ultra-sons donnent des résultats d'autant meilleurs que les conditions préférées sont remplies.

La cuve à ultra-sons utilisée dans l'article précité, pourrait être la cause de cet échec.

En effet, pour obtenir un bon résultat, il convient que la puissance d'ultra-sons émise directement par la paroi assurant cette émission soit au moins égale à 20 avantageusement à 50W/cm$^2$, plus avantageusement à 100W/cm2, de préférence à 200 W/cm2.

Ainsi l'existence d'une valeur limite inférieure de la puissance est d'autant plus importante lorsqu'on utilise une cuve à ultra-sons dans laquelle l'ultra-son n'est pas transmis directement par une sonde mais au travers de la paroi du récipient et du milieu dans lequel il baigne, dans lequel intervient la réaction.

Dans la présente description on considère que la transmission directe lorsque la partie active de la soude n'est pas séparée du milieu réactionnel par un liquide.

Il n'y a pas de valeur supérieure limite, toutes les puissances mises sur le commerce conviennent pour activer cette réaction d'échange. Il convient toutefois de choisir cette puissance de manière que le récipient où a lieu la réaction ne soit pas fragilisé par les ultra-sons.

Les fréquences utilisables sont celles des appareils du commerce, c'est-à-dire que la fréquence des ultra-sons est avantageusement comprise entre 10 et 100 KHz, de préférence entre 15 et 50. Toutefois certaines fréquences donnent de résultats très significativement meilleurs. Ces fréquences préférées dépendent du milieu réactionnel et des conditions d'emploi.

Ces fréquences préférées, correspondent à celles de résonnance du milieu dans les conditions de l'expérience.

Ces fréquences sont aisément déterminables par l'homme de métier. Ce sont les fréquences correspondants à des maxima d'absorbtion énergétique par le milieu.

Un simple balayage de fréquence, de préférence à puissance maximum, permet de déterminer les pics d'absorbtion énergétique.

Cette recherche de la meilleure absorbtion n'est pas critique, en effet les appareils usuels émettent un spectre suffisamment large pour qu'au moins une fréquence de résonnance soit couverte. Si, en revanche, on utilise un générateur donnant un ultra-son très pur, une telle recherche sera utile, voire indispensable pour obtenir pleinement les avantages désirés.

Aussi il est hautement souhaitable que le spectre d'ultra-son émis contienne partiellement au moins une fréquence de résonnance.

L'utilisation d'une telle technique a permis de réaliser, à des températures comprises entre 100 et 200° C, de préférence entre 100 et 150° C, avec de très bons rendements des réactions qui n'avaient lieu avec les techniques ordinaires qu'à des températures très élevées, soit aux alentours de 250° C.

Il convient de noter que le volume dans lequel la réaction est accélérée est relativement réduit et est déterminé par un cylindre défini par la surface efficace de la sonde et une génératrice, normale à la surface de la sonde dont la longueur n'excède pas 10 avantageusement 5 cm environ. Il apparaît que plus le mélange réactionnel est proche de la sonde, plus rapide est la réaction. On peut donc considérer comme volume efficace, un volume définit par la surface de la sonde, et une génératrice d'une taille comprise entre 1 et 5 cm.

Les techniques d'agitation ou le génie chimique pour mettre en oeuvre la présente invention devra tenir compte de ce volume efficace, et faire en sorte que l'essentiel du mélange réactionnel passe de nombreuses fois dans ce volume efficace.

Lorsque l'opération est menée en discontinu (c'est-à-dire par lots) le rapport entre le volume du milieu réactionnel et le volume efficace (ou le rapport de temps) est au plus égal à environ 20 avantageusement 10 de préférence 5 ; lors d'opération en continu ou par impulsion, tiendra compte du temps de séjour et/ou de l'impulsion dans le volume efficace.

Le procédé selon la présente invention atteint sa meilleure efficacité lorsque les conditions ci-après sont réunies.

De préférence la réaction a lieu dans un solvant aprotique dipolaire et met en oeuvre un milieu (il est à noter que ce milieu apporte en lui-même une amélioration significative même sans ultra-sons) comportant:

   – un solvant, ou réactif, aprotique dipolaire ;

   – une phase solide constituée au moins partiellement de fluorure alcalin, et éventuellement au moins un cation promoteur de la réaction.

Avantageusement, le taux de solide du milieu défini en tant que masse de matière solide rapporté à la masse totale est au moins égale à 2/5 avantageusement à 1/2, de préférence à 2/3, soit rapporté au mélange réactionnel respectivement 1/5, 1,4, 1/3.

Avantageusement, le réactif comporte à titre de promoteur un cation alcalin plus lourd que le potassium. Ce cation alcalin peut être introduit sous forme d'un halogénure et pas nécessairement sous forme d'un fluorure. En général, on introduit le cation alcalin sous forme d'un chlorure. La teneur en cation alcalin est avantageusement comprise entre 1 à 5 %, de préférence entre 2 à 3 % en mole du fluorure alcalin utilisé.

Le réactif peut comporter à titre de promoteur des agents souvent qualifiés de transfert de phase et qui sont des "oniums", c'est-à-dire les composés dont le nom contient l'affixe "onium". Les oniums représentent en général de 1 à 10 %, de préférence de 2 à 5 % en mole du fluorure alcalin.

Parmi ces oniums, les réactifs préférés sont les tétra alcoyl ammonium de 4 à 12 atomes de carbone, de préférence de 4 à 8 atomes de carbone. Le tétra alcoyl ammonium est en général du tétra méthyl ammonium. Le terme alcoyle est pris dans l'acception "lato sensu" donnée par le dictionnaire de Chimie DUVAL.

Toutefois, les tétra alcoyl ammonium présentent l'inconvénient d'être peu stables à une température supérieure à environ 150° C. C'est un des avantages de la présente invention dans ces mises en oeuvres préférées que de permettre des réactions à des températures inférieures ou voisines de cette valeur, ce qui permet l'utilisation des ammoniums notamment le tétra méthyl ammonium.

La quantité de cation tétra alcoyl ammonium est en général comprise entre 1 à 10 %, de préférence entre 2 à 5 % de la masse du fluorure alcalin.

Le solvant aprotique dipolaire utilisable doit avoir un moment dipolaire significatif. Ainsi, sa constante diélectrique relative ε est au moins égale à environ 10 (les zéros de position ne sont pas considérés comme des chiffres significatifs dans la présente description, à moins que cela ne soit précisé autrement). De préférence, l'ε est inférieur ou égal à 100 et supérieur ou égal à 25.

Il a pu être montré au cours de la présente étude que les meilleurs résultats étaient obtenus lorsqu'on utilisait des solvants aprotiques dipolaires qui présentaient un indice c donneur compris entre 10 et 50. Ledit indice c donneur étant comme le $\Delta H$ (variation d'Enthalpie) exprimé en kilo calories (Kcal) de l'association dudit solvant aprotique dipolaire avec le pentachlorure d'antimoine dans le chlorure de méthylène.

Les oniums sont choisis dans le groupe des cations formés par les éléments des colonnes V B et VI B (tels que définis dans le tableau de la classification périodique des éléments publiés au supplément au Bulletin de la Société Chimique de France en Janvier 1966) avec respectivement 4 ou 3 chaînes hydrocarbonnées. On ne s'écartera pas de l'invention en utilisant les dérivés dans lesquels une chaîne, voire plusieurs chaînes sont remplacées par une ou des insaturations dès lors qu'ils sont considérés comme stables dans les conditions réactionnelles.

Ainsi que cela a été dit précédemment, la granulométrie joue un rôle relativement peu important dans la cinétique dès lors que la granulométrie n'est pas trop fine, c'est-à-dire lorsque la majorité des particules est supérieure à un micron et que la majorité des particules est inférieure à 250 microns.

Toutefois, il a été montré que la granulométrie avait une influence sur la cinétique. Ainsi, il est souhaitable que ledit solide en supension présente une granulométrie tel que son $d_{90}$ (défini en tant que la maille laissant passer 90 % en poids du solide) est au plus égale à 100 micromètres, avantageusement au plus égale à 50 µm, de préférence au plus égale à 20µm.

La limite inférieure est avantageusement caractérisé telle que le $d_{10}$ dudit solide en supension est au moins égal à 0,1 µm, de préférence au moins égal à 1 µm.

En général, le rapport entre ledit fluorure alcalin et ledit substrat est compris entre 1 et 1,5, de préférence aux alentours de 1,25 de la stoechiométrie. Ce rapport n'est toutefois en général pas critique dès lors qu'il permet une agitation convenable du réactif.

Selon la présente invention, il est souhaitable que la teneur en eau du réactif soit au plus égale à environ 2 % de préférence 1 % par rapport à la masse du réactif (rapport en masse).

L'agitation est avantageusement menée de manière que au moins 80 % de préférence au moins 90 % des solides soient maintenus en suspension par l'agitation. En général, l'agitation due aux ultra-sons suffit dans la zone où ils sont actifs.

En fait, il est souhaitable que la plus grande partie des solides soient en suspension dans le milieu réactionnel.

Ainsi l'agitation, pour répondre à cette contrainte, ne doit être ni trop forte ni trop faible. En effet, trop forte, elle risque de plaquer par des effets de cyclone une partie trop importante des solides contre la paroi du réacteur. Dans le deuxième cas, l'agitation sera incapable de faire entrer en suspension les solides issus de la réaction et les solides utilisés comme source de fluor.

Cet échange peut être réalisé par tout fluorure connu de l'homme de métier pour être un bon réactif d'échange. On préfère toutefois les cations alcalins de rang au moins égal à celui du potassium. On peut également utilisé des cations non alcalins ayant des propriétés équivalentes, comme par exemple les fluorures de tétra-alcoyl ammonium. Une des mises en oeuvre de la présente invention peut être réalisée par la séquence d'opérations suivantes :

    a) Mélange des réactifs.

    b) Soumission du mélange obtenu au a) à au moins une opération de réaction sous ultra-son.

    c) Séparation des phases liquides et solides.

    d) Traitement de la phase liquide obtenue de manière à récupérer le produit final, d'une part, et le solvant contenant le produit de départ n'ayant pas réagi.

    e) Recyclage du solvant et du produit de départ n'ayant pas réagi vers a).

    f) Etape optionnelle de traitement de la phase solide provenant de l'étape c) pour régénérer le fluorure alcalin et recyclage à l'étape a).

Il convient de signaler que l'utilisation d'ultra-sons dégage une quantité d'énergie importante au sein du milieu réactionnel, cette énergie se substitue en tout ou partie à l'énergie de chauffage usuellement nécessaire.

Il convient de souligner que la présente invention permet un progrès très significatif pour l'utilisation des solvants.

Lorsque l'on traite des molécules lourdes (masse moléculaire $\cong 200$) ou particulièrement lourdes (masse moléculaire $\cong 500$), ou pour toute autre raison, il peut être avantageux qu'une partie des solides du réactif, et donc du milieu réactionnel, soit constitué d'un matériau inerte de granulométrie répondant aux contraintes spécifiées ci-dessus. En effet, la sonification affranchit l'homme de métier des contraintes de résistance à des températures élevées et par conséquent de point d'ébullition élevé pour les solvants utilisés lors de l'échange du fluor avec un halogène de rang plus élevé, il est préférable de ne pas travailler à une température très proche de la température du milieu au moins 10° C d'écart.

Cela ouvre des perspectives particulièrement intéressantes pour la séparation des solvants d'avec les réactifs produits de départ et produits d'arrivée, et facilite le recyclage des mélanges réactionnels.

Il peut ainsi être envisagé de régénérer la source de fluor lorsque le cation associé est coûteux, comme par exemple dans le cas du cesium.

On peut utiliser cette technique pour échanger un halogène ou équivalent tant sur un carbone aliphatique que sur un carbone aromatique.

Lorsque le radical est un aryle, il est de préférence appauvri en électrons et a une densité électronique au plus égale à celle du benzène, de préférence au plus voisine de celle d'un halobenzène. Cet appauvrissement peut être dû à la présence dans le cycle aromatique (en général hexagonal) d'un hétéro atome, comme par exemple à titre d'enseignement dans la pyridine, la quinoléine.

Bien évidemment, l'appauvrissement électronique peut être provoqué aussi par des groupes électro-attracteurs ; la pauvreté en électrons peut être dûe à ces deux causes.

Ainsi ledit aryle porte avantageusement au moins un substituant sur le même noyau que celui portant le chlore ou équivalent ledit au moins un substituant étant de préférence choisi parmi les groupes attracteurs par effet inductif ou par effet mésomère tel que défini dans l'ouvrage de référence en Chimie Organique "Advanced Organic Chemistry" par M.J. MARCH, 3ième édition, Editeur WILLEY, 1985, cf. notamment P. 17 et 238, à l'exception des groupes susceptibles d'inter agir avec les ions fluorures (essentiellement les groupes porteurs d'hydrogène susceptible de donner des liaisons hydrogène) comme exemple de groupe attracteur on peut citer: $NO_2$, $CF_3$, CN, COX (X = Cl, Br, F,OR), CHO, Cl, Br. On peut également exprimer cette référence en disant que la constante de Hammett est au moins égal à 0,1 de préférence à 0,2, plus précisément $\Sigma \sigma_p$ supérieur ou égal aux valeurs ci-dessus, dans cette formule, qui emprunte au symbolisme mathématique, $\Sigma$ (en toute vigueur, il conviendrait d'écrire selon le symbolisme $\Sigma \sigma_p i$ où n est le nombre de sommet du noyau considéré) signifie que l'on fait la somme arithmétique des constantes de Hammett de tous les substituants du noyau considéré : comme l'effet du substituant sur le noyau n'est pas complètement uniforme sur les diverses positions, et que par voie de conséquence la constante de Hammett varie (légèrement) selon la position du sommet concerné par rapport au substituant, on choisit pour faire le calcul, la constante $\sigma_p$ de la position para lorsque le substituant est seul. Pour plus de détails sur les constantes de Hammett, on peut se référer à divers ouvrages et notamment à l'ouvrage à caractère général ci-dessus (pages 242 à 250) qui en outre donnent les valeurs de $\sigma_p$ pour les groupes les plus courants (page 244). Il n'y a pas de valeur supérieure pour $\sigma_p$ sinon que cela dépasse rarement 2 et très rarement 3.

Le procédé pour la synthèse de fluorure d'aryle à partir de composés halogénés par réaction d'échange entre halogènes, consistant à mettre en contact ledit composé halogéné avec un réactif comportant :
– un solvant aprotique dipolaire ;
– une phase solide constituée au moins partiellement de fluorure alcalin;
– éventuellement un cation promoteur ;
– le taux de solide étant au moins égal à 1/5, avantageusement à 1/4, de préférence à 1/3, avec la condition que lorsque simultanément le solvant aprotique dipolaire est du sulfolane, et qu'il n'y a pas de cation promoteur et que le rapport molaire entre le substrat et le sulfolane est supérieur à 0,5 et à 1, le taux de solide est respectivement au moins égal à 1/3 et 2/5, donne de bons résultats même sans ultra-sons.

Ainsi que mentionnée précédemment, l'agitation est avantageusement menée de manière que au moins 90 % des solides soient maintenus en suspension par l'agitation. En fait, il est souhaitable que la plus grande partie des solides soient en suspension dans le milieu réactionnel. Ainsi, l'agitation, pour répondre à cette contrainte, ne doit être ni trop forte ni trop faible. En effet, trop forte, elle risque de plaquer par des effets de cyclone, une partie trop importante des solides contre la paroi du réacteur. Dans le deuxième cas, l'agitation sera incapable de faire entrer en suspension les solides issus de la réaction et les solides utilisés comme source de fluor. Des dispositifs tels que ceux décrits dans la demande de brevet Européen publiée sous le N° 115 559 Al permettent une bonne agitation avec peu de consommation d'énergie.

Les exemples non limitatifs suivants illustrent l'invention.

**EXEMPLE 1**

Dans un réacteur de 70 ml, on charge sous atmosphère d'azote :
– 17,7 g (306 mmoles) de fluorure de potassium
– 1,3 g (12 mmoles) de chlorure de tetraméthylammonium
– 17,0 g de diméthylsulfoxyde
– 19,6 g (102 mmoles) de dichloro- 2,4 nitrobenzène.
Le mélange réactionnel est traité par ultra-sons, la température étant maintenue à 130° C, pendant 30 minutes.
Après filtration, l'analyse du brut réactionnel fournit les résultats suivants :
– conversion du dichloro-2,4 nitrobenzène      :      67,1 %
– rendement en fluoro chloro nitrobenzènes      :      52 %
– rendement en difluoro-2,4 nitrobenzène      :      13,9 %

**Exemple 2**

Le même protocole que dans l'exemple 1 est utilisé. La masse réactionnelle n'est pas soumise à l'action des ultra-sons mais chauffée à 130° C pendant 30 minutes :
– conversion du dichloro-2,4 nitrobenzène      :      43,8 %
– rendement en fluoro chloro nitrobenzènes      :      34,3 %
– rendement en difluoro-2,4 nitrobenzène      :      4,0 %

**Exemples 3 et 4**

Deux exemples comparatifs identiques aux cas des exemples 1 et 2 sont réalisés pour utilisation du mélange fluorant KF + CsF (3 %) dans le sulfolane à 130° C pendant trente cinq minutes :

| N° d'exemple | ultra-son | conversion (%) | rendement DiF24NB (%) | rendement en monofluorés |
|:---:|:---:|:---:|:---:|:---:|
| 3 | OUI | 38,4 | 3,4 | 34 |
| 4 | NON | 3,2 | 0 | 2,3 |

**Exemple 5**

Dans un réacteur de 30 ml fermé par un septum, on charge sous atmosphère d'azote :
– 4,06 g KF (70 Mmoles),
– 0,3 g Me$_4$NCl (2,7 Mmoles),
– 5,63 g dichloro-2,4 nitrobenzène (29,3 Mmoles),
– 8,16 g de diméthylsulfoxyde.
Le mélange est traité par ultra-sons par cycle. Pour chaque cycle d'1 seconde : 75 % de ce temps, les ultra-sons sont au repos.
La température est maintenue à 130°C pendant 30 minutes.
Conversion du dichloro-2,4 nitrobenzène      :      84 %
Rendement en fluorochloronitrobenzène      :      47 %
Rendement en difluoro-2,4 nitrobenzène      :      34 %.

**Exemple 6**

Dans un réacteur de 30 ml fermé par un septum, on charge sous atmosphère d'azote :
– 8,14 g de diméthylsulfoxyde,

– 0,3 g Me$_4$ NCl (2,7 Mmoles),
– 4,03 g de KF (70 Mmoles).

Ce mélange est préalablement traité aux ultra-sons pendant 10 minutes en maintenant une température de 50°C. On arrête les ultra-sons puis on injecte rapidement 5,7 g (29,7 Mmoles) de dichloro-2,4 nitrobenzène en maintenant une température de 130°C par un chauffage traditionnel.

Après 30 minutes, on obtient :

| | | |
|---|---|---|
| Conversion du dichloro-2,4 nitrobenzène | : | 79 % |
| Rendement en fluorochloronitrobenzène | : | 49 % |
| Rendement en difluoro-2,4 nitrobenzène | : | 30 % |

## Exemple 7

Un réacteur de 250 ml cylindrique en verre est surmonté d'une agitation à ancre, d'un réfrigérant ascendant, d'une gaine thermométrique, d'un tube plongeant et d'un tube arrivée.

Ces deux tubes sont reliés à un second réacteur de 30 ml en acier inox surmonté d'une sonde ultra-sonique, ceci par l'intermédiaire d'une pompe servant à faire circuler le mélange réactionnel entre les 2 réacteurs à un débit moyen de 30 l/h.

Dans le réacteur en verre, on charge :
– 150 g de diméthylsulfoxyde
– 75 g de KF (1,29 mole)
– 99,3 g de dichloro-2,4 nitrobenzène (0,52 mole)
– 4,8 g de Me$_4$NCl.

Le mélange réactionnel est chauffé à 130°C, agité, mis en circulation dans les deux réacteurs et traité par ultra-sons par impulsion de 50 %. La durée de l'essai est de 1h30 minutes.

| | | |
|---|---|---|
| Conversion du dichloro-2,4 nitrobenzène | : | 99 % |
| Rendement en fluorochloronitrobenzène | : | 18,8 % |
| Rendement en difluoro-2,4 nitrobenzène | : | 73,5 % |

## Exemple 8

Un essai comparatif effectué dans le réacteur de 250 ml mais seulement chauffé d'un façon traditionnelle, on obtient après 1h30 à 130°C les résultats suivants :

| | | |
|---|---|---|
| Conversion du dichloro-2,4 nitrobenzène | : | 83 % |
| Rendement en fluorochloronitrobenzène | : | 35,2 % |
| Rendement en difluoro-2,4 nitrobenzène | : | 39,5 % |

## Exemple 9

Dans un réacteur de 50 ml, on charge sous atmosphère d'azote :
– 5,8 g de KF (100 Mmoles),
– 0,43 g de Me$_4$ NCl (0,4 Mmoles),
– 7,75 g de dichloro-2,4 nitrobenzène (40,3 Mmoles),
– 11,6 g de diméthylsulfoxyde.

Le mélange réactionnel est traité par ultra-sons en optimisant la fréquence ultra-sons en fonction de la fréquence de résonnance du système. Ainsi on fait agir pendant 30 secondes à une fréquence de 20 350 Hz à 130°C et on obtient :

| | | |
|---|---|---|
| Conversion du dichloro-2,4 nitrobenzène | : | 81,5 % |
| Rendement en chlorofluoro nitrobenzène | : | 48 % |
| Rendement en difluoro-2,4 nitrobenzène | : | 31 % |

## Exemple 10

Dans un réacteur de 50 ml, on charge sous atmosphère d'azote :
– 5,8 g de KF,
– 0,43 g de Me$_4$ NCl,
– 7,75 g de dichloro-2,4 nitrobenzène,
– 11,6 g de diméthylnitrobenzène sulfoxyde.

Le mélange réactionnel est traité par ultra-sons à 150°C pendant 30 minutes. On obtient :

Conversion du dichloro-2,4 nitrobenzène    :    99 %
Rendement en chlorofluoro nitrobenzène    :    15 %
Rendement en difluoro-2,4 nitrobenzène    :    80 %

**Exemple 11**

Dans un réacteur de 30 ml, on charge sous atmosphère d'azote :
- 2,9 g de KF (50 Mmoles),
- 0,75 g de $Ph_4$ PCl (2 Mmoles),
- 4 g de chloro-4 nitrobenzène (25,5 Mmoles),
- 12 g de diméthylsulfoxyde.

Le mélange réactionnel est traité par ultra-sons à 170°C pendant 1 heure.

Conversion du chloro-4 nitrobenzène    :    60 %
Rendement en fluoro-4 nitrobenzène    :    54 %

**Exemple 12**

Dans le même réacteur, on charge d'une façon identique les réactifs mais on chauffe l'ensemble par un chauffage traditionnel à 170°C pendant 1heure sous agitation magnétique.

On obtient :

Conversion du chloro-4 nitrobenzène    :    39 %
Rendement en fluoro-4 nitrobenzène    :    34 %

Les exemples montrent l'intérêt du milieu selon l'invention même sans utilisation d'ultra-sons.

**Exemple 13**

Dans un réacteur de 1l, préchauffé à 50°C et placé sous atmosphère d'azote, est chargé la suspension de fluorure de potassium (174 g, 3 moles) de chlorure de tétraméthyle ammonium (13 g, 0,12 mole) dans le diméthylsulfoxyde (176 g, 2,26 moles). Cette suspension a été préalablement broyé pendant 30 minutes dans un broyeur à billes Netzch, type LME 1. La suspension avant broyage avait les caractéristiques granulométriques suivantes :
- Taille moyenne : 65 μm, $d_{10}$ : 24 μm, $d_{90}$ : 140 μm.

Après broyage, les caractéristiques granulométriques sont :
- Taille moyenne 11μm, $d_{10}$ : 2μm, $d_{90}$ : 22μm.

    . Sauf indication contraire, le fluorure de potassium utilisé contient moins de 100 ppm d'eau et le solvant environ 50 ppm d'eau.

    . On introduit ensuite le dichloro-2,4 nitrobenzène (230,4 g, 1,2 moles). L'agitation du milieu est telle que l'ensemble du solide est maintenu en suspension. Après 3 h 30 de chauffage à 130° C, l'analyse du mélange réactionnel montre que la conversion du dichloro-2,4 nitrobenzène est complète, que le rendement en difluoro 2,4 nitrobenzène est de 92 %, et que le rendement en fluoro chloro nitrobenzène est de 8 %. Ce mélange des isomères monofluorés monochlorés majoritairement fluoro 4.

Le réacteur est alors refroidi à 30° C, le milieu réactionnel est dilué à l'aide de 100 ml de chlorure de méthylène. Le mélange est agité 10 minutes et transvasé sur un verre fritté. Le réacteur et le précipité sont rincés deux fois par 100 ml de chlorure de méthylène. Le filtrat est récupéré et, le chlorure de méthylène est évaporé à l'aide d'un évaporateur rotatif ( temperature bain = 70° C, p = 100 mm Hg). Le DMSO est éliminé par lavage à l'eau et le résidu est distillé. On obtient ainsi 173,6 g de difluoro-2,4 nitrobenzène (Rendement 91 % pureté > 99 %).

**Exemple 14**

Une procédure analogue à celle décrite dans l'exemple 1 mais à partir d'un fluorure de potassium non broyé conduit après 4 h 30 de chauffage à 130° C aux résultats suivants :
- Conversion du dichloro-2,4 nitrobenzène    =    99,7 %.
- Rendement en difluoro-2,4 nitrobenzène    =    90,4 %.
- Rendement en fluoro chloro nitrobenzènes    =    9,7 %.

Après traitement et distillation, on obtient 173,9 g de produit (rendement en difluoro-2,4 nitrobenzène 87,4 %, pureté 96,3 %).

**Exemple 15**

Un procédure analogue à celle décrite dans l'exemple 1, à partir d'un fluorure de potassium non broyé et de 378 g (4,85 moles) de diméthylsulfoxyde conduit après 6 h de chauffage à 130° C aux résultats suivants :
- Conversion du dichloro-2,4 nitrobenzène = 100 %.
- Rendement en difluoro-2,4 nitrobenzène = 93,4 %.
- Rendement en fluoro chloro nitrobenzènes = 1 %.

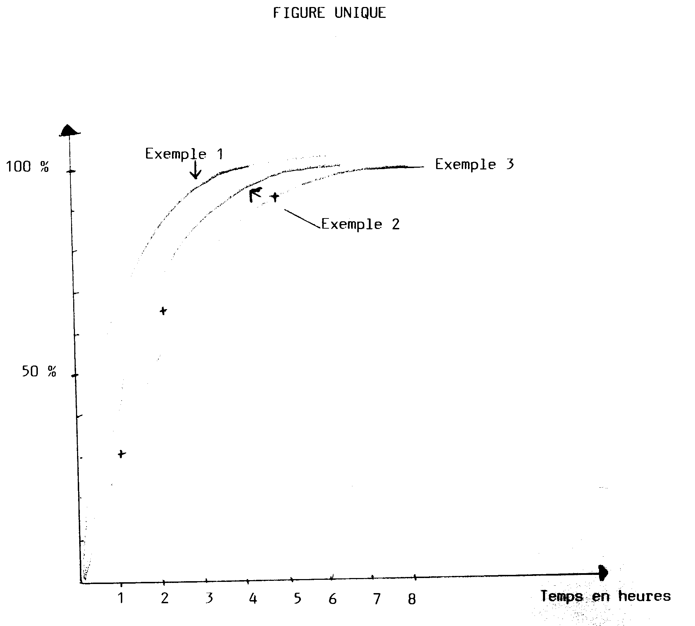

La comparaison cinétique de ces trois essais est réalisée sur la courbe suivante donnant chaque cas le rendement en difluoro-2,4 nitrobenzène en fonction du temps (cf. Figure Unique).

**Exemple 16**

La réaction est réalisée dans un réacteur de 100 ml, placé sous atmosphère d'azote, dans lequel on introduit successivement :

28 g de fluorure de potassium (0,048 mole),

1,8 g de fluorure de césium (12 mmoles),

28 g de dichloro-2,4 nitrobenzène (0,146 mole) et 60 g de sulfolane.

Le mélange réactionnel est chauffé à 180° C en maintenant une agitation telle que le solide soit maintenu en suspension. Après 6 h de chauffage les résultats suivants sont obtenus :
- Conversion du dichloro-2,4 nitrobenzène = 100 %
- Rendement en fluoro-2,4 nitrobenzène = 95 %
- Rendement en fluoro chloro nitrobenzènes = 5 %

**Exemple 17**

Dans un réacteur de 50 ml placé sous atmosphère d'azote, on charge 2,4 g de fluorure de potassium (41,4 mmoles), 3,9 g de dichloro-2,4 nitrobenzène (20,5 mmoles) et 6 g de sulfolane.

Le mélange est chauffé à 180° C pendant 11 heures. Le mélange réactionnel est ensuite filtré et le filtrat analysé par CPG :
- Conversion du dichloro-2,4 nitrobenzène = 85,5 %
- Rendement du dichloro-2,4 nitrobenzène = 29,0 %
- Rendement en fluoro chloro nitrobenzènes = 44,7 %

**Exemples 18 à 20**

Le protocole de l'exemple 5 est repris avec addition d'une quantité catalytique d'eau en début d'essai.

| N°d'exemple | % H$_2$O (poids) | conversion DiCl2,4NB(%) | rendement DiF2,4NB (%) | rendement en monofluorés |
|---|---|---|---|---|
| 18 | 0,4 | 84,2 | 26,8 | 47,5 |
| 19 | 1,1 | 83,1 | 25,5 | 45,3 |
| 20 | 2,6 | 41,8 | 4,5 | 27,0 |

**Exemple 21**

Dans un réacteur de 50 ml placé sous atmosphère d'azote, on charge 2,6 g de fluorure de potassium (45,2 mmoles), 2,96 g de dichloro-2,4 nitrobenzène (5,4 mmoles) et 6,2 g de DMSO.

Le mélange est chauffé à 130° C pendant 6 h. Le mélange réactionnel est ensuite filtré et le filtrat analysé par CPG :
- Conversion du dichloro-2,4 nitrobenzène       :       71 %
- Rendement en difluoro-2,4 nitrobenzène       :       21,8 %
- Rendement en fluoro-4 chloro-2 nitrobenzène       :       11,9 %
- rendement en fluoro-2 chloro-4 nitrobenzène       :       39,6 %.

## Exemple 22 à 24

Le protocole de l'exemple 9 est repris avec addition d'une quantité catalytique de divers oniums :

| N° ex. | Onium | Onium (% molaire) KF | Conversion DiCl$_2$4NB (%) | Rendement DiF24NB (%) | Rendement en monofluorés (%) |
|---|---|---|---|---|---|
| 22 | Me$_4$ NCl | 2,15 | 100 | 93,1 | 1,8 |
| 23 | Ph$_3$SCl | 3,87 | 8,9 | 65,6 | 18,6 |
| 24 | Ph$_4$ AsCl | 3,92 | 100 | 66,6 | 0,6 |
| 25 | Bz Me$_3$ NBr | 4,1 | 99,9 | 76,8 | 9,2 |
| 26 | BzMe$_3$ NHF$_2$ | 4,4 | 98,7 | 71,5 | 18,0 |

## Exemples 27 à 29

Le protocole de l'exemple 10 est reproduit pour divers solvants:

| N° d'exemple | SOLVANT | Conversion DiCl$_2$4NB (%) | Rendement DiF24NB (%) | Rendement en monofluorés (%) |
|---|---|---|---|---|
| 27 | Sulfolane | 100 | 84,1 | 3,3 |
| 28 | DMF | 99,5 | 78,3 | 15,0 |
| 29 | DMAC | 97,2 | 55,5 | 25,5 |

## Exemple 30

Le protocole de l'exemple 5 est reproduit par chauffage à 180° C pendant 6 heures du mélange de fluorure de potassium, (61,5 mmoles, 3,6 g) dichloro-2,4 nitrobenzène (20,5 mmoles, 3,9 g) et sulfolane (6,2 g) :

– Conversion du dichloro-2,4 nitrobenzène    =    81,7 %
– Rendement en difluoro-2,4 nitrobenzène    =    47,7 %

## Exemples de 31 à 37

Le protocole de l'exemple 30 est, reproduit avec addition d'une quantité catalytique de fluorure de césium, chlorure de césium ou fluorure de rubidium

| N° d'exemple | Catalyseur | Catalyseur (% molaire) KF | Conversion DiCl2,4 NB (%) | Rendement DiF2,4 NB (%) | Rendement en monofluorés (%) |
|---|---|---|---|---|---|
| 31 | CsF | 0,1 | 85,4 | 29,5 | 45,2 |
| 32 | CsF | 0,9 | 99,5 | 71,9 | 12,1 |
| 33 | CsF | 1,5 | 99,3 | 72,4 | 15 |
| 34 | CsF | 2,5 | 100 | 95,3 | 0 |
| 35 | CsF | 5 | 100 | 96,4 | 0 |
| 36 | CsCl | 1 | 98,6 | 64,9 | 3,1 |
| 37 | RbF | 4,7 | 98,4 | 48,2 | 7,0 |

## Exemple 38

Dans un réacteur de 50 ml placé sous atmosphère d'azote, on charge:
– 3 g de fluorure de potassium (51,7 mmoles)
– 7,8 g de dichloro-2,4 nitrobenzène (40,6 mmoles) et 6g de sulfolane.
Le mélange réactionnel est chauffé 6h 0 180° C. Après refroidissement le mélange réactionnel est filtré et le filtrat analysé par CPG :
– conversion du dichloro-3,4 nitrobenzène    =    4,8 %
– rendement en difluoro-3,4 nitrobenzène    =    4,4 %

## Exemples 39 à 41

Le protocole de l'exemple 38 est répété avec diverses conditions réactionnelles :

| N° Ex. | Conditions réac-tionnelles | Cata-lyseur | Catalyseur KF (%) | Conversion (%) | Rendement (%) |
|---|---|---|---|---|---|
| 39 | DM30 - 130°C- 6h | - | - | 2,2 | 2,0 |
| 40 | DM30 - 170°C- 6h | Mc4NCl | 3,9 | 64 | 61,2 |
| 41 | Sulfolane-180°C-6h | CsF | 2,6 | 28,9 | 27,5 |

**Revendications**

**1).** Procédé de synthèse de composés organo fluorés par l'échange avec des fluorures caractérisé par le fait que l'échange est réalisé sous l'action d'ultra son.

**2)** Procédé selon la revendication 1 caractérisé par le fait que les ultra sons sont transmis directement par la sonde dans le milieu réactionnel.

**3)** Procédé selon l'une des revendications 1 et 2 caractérisé par le fait que la puissance transmise au milieu par l'intermédiaire de la surface active de la sonde est au plus égale à 50 Watts/cm2, avantageusement à 100W/cm2 et de préférence à 200W/cm2.

**4)** Procédé selon l'une des revendications 1 à 3 caractérisé par le fait que la fréquence des ultra-sons est comprise entre 10 et 100 KHz.

**5)** Procédé selon l'une des revendications 1 à 4 caractérisé par le fait que la réaction est réalisée à une température comprise entre 100 et 200° C, de préférence entre 100 et 150° C.

**6)** Procédé selon l'une des revendications 1 à 5, caractérisé par le fait que l'émission de l'ultra-son est réalisée dans le récipient de telle manière que les particules de fluorure passent au travers d'un volume défini par la surface de la sonde et une génératrice d'une taille comprise entre 1 à 5 cm.

**7)** Procédé selon l'une des revendications 1 à 6 caractérisé par le fait que le fluorure est un fluorure alcalin ; le nombre atomique du métal alcalin étant au moins égale à celui du potassium.

**8)** Procédé selon l'une des revendications 1 à 8 caractérisé par le fait que la réaction d'échange a lieu dans un solvant aprotique dipolaire caractérisé par le fait que le taux de solide défini en tant que masse de matière solide rapporté à la masse totale est au moins égale à 1/5 avantageusement à 1/4, de préférence à 1/3.

**9)** Procédé selon l'une des revendications 1 à 8 caractérisé par le fait que le milieu réactionnel comporte en outre un cation alcalin plus lourd que le potassium.

**10)** Procédé selon l'une des revendications 1 à 9 caractérisé par le fait que ledit cation alcalin représente de 1 à 5 % en poids de la masse dudit fluorure alcalin de préférence de 2 à 3 %.

**11)** Procédé selon l'une des revendications 1 à 10 caractérisé par le fait que le milieu réactionnel comporte en outre un onium.

**12)** Procédé selon la revendication 11 caractérisé par le fait que ledit onium représente 1 à 10 %, de préférence 2 à 5 % en poids du fluorure alcalin.

**13)** Procédé selon l'une des revendications 1 à 10 caractérisé par le fait que le mélange réactionnel comporte en outre un cation tétra-alcoyl ammonium de 4 à 12 atomes de carbone, de préférence de 4 à 8 atomes de carbone.

**14)** Procédé selon l'une des revendications 1 à 13 caractérisé par le fait que ledit solvant aprotique dipolaire présente une constante diélectrique relative $\varepsilon$ supérieure ou égale à 10, de préférence inférieure ou égale à 100 et supérieure ou égale à 25.

**15)** Procédé selon l'une des revendications 1 à 14, caractérisé par le fait que le solvant aprotique di polaire présente un indice c donneur compris entre 10 et 50, ledit indice c donneur étant défini comme le $\Delta H$ exprimé en kilo calories de l'association dudit solvant aprotique di polaire avec le penta chlorure d'antimoine.

**16)** Procédé selon l'une des revendications 11 à 15 caractérisé par le fait que les oniums sont choisis dans le groupe des cations formés par les éléments des colonnes V B et VI B (tels que définis dans le tableau de la classification périodique des éléments publiés au supplément au Bulletin de la Société chimique de France en Janvier 1966) avec respectivement 4 ou 3 chaînes hydrocarbonnées.

**17)** Procédé selon les revendications 1 à 16 caractérisé par le fait que ledit solide en suspension présente une granulométrie tels que sont $d_{80}$ (défini en tant que la maille laissant passer 80 % en poids du solide) est au plus égale à 100 micromètres, avantageusement au plus égale à 50 $\mu$m de préférence au plus égale à 20 $\mu$m.

**18)** Procédé selon l'une des revendications 1 à 17 caractérisé par le fait que le $d_{20}$ dudit solide en suspension est au moins égal à 0,1 $\mu$/m, de préférence au moins égal à 1 $\mu$m.

**19)** Procédé selon l'une des revendications 1 à 18, caractérisé par le fait qu'il comporte en outre ledit composé chloré, le rapport entre ledit fluorure alcalin et ledit substrat étant compris entre 1 et 1,5, de préférence aux alentours de 1,25.

**20)** Procédé selon l'une des revendications 1 à 19, caractérisé par le fait que l'agitation est menée de manière que au moins 80 % de préférence des solides soient en suspension.

**21)** Procédé selon l'une des revendications 14 et 15 caractérisé par le fait que la réaction est menée à une température comprise entre 100 et 150° C.

**22)** Procédé selon l'une des revendications 1 à 21 caractérisé par le fait qu'il comporte les étapes suivantes :

a) Mélange des réactifs.

b) Soumission du mélange obtenu au a) à au moins une opération de réaction sous ultra-son.

c) Séparation des phases liquides et solides.

d) Traitement de la phase liquide obtenue de manière à récupérer le produit final, d'une part, et le solvant contenant le produit de départ n'ayant pas réagi.

e) Recyclage du solvant et du produit de départ n'ayant pas réagi a).

f) Etape optionnelle de la phase solide provenant de l'étape c) pour régénérer le fluorure alcalin et recyclage à l'étape a).

**23)** Réactif utile pour la synthèse sous ultra-sons de fluorure d'aryle à partir de composés chlorés par réaction d'échanges entre halogènes comportant :

– un solvant aprotique dipolaire ;

– une phase solide constituée au moins partiellement de fluorure alcalin ;

caractérisé par le fait que le taux de solide défini en tant que masse de matière solide rapporté à la masse totale est au moins égale à 2/5 avantageusement à 2/4, de préférence à 2/3.

**24)** Réactif selon la revendication 23 caractérisé par le fait qu'il comporte en outre au moins un onium et/ou un cation alcalin plus lourd que le potassium.

FIGURE UNIQUE

Office européen
des brevets

# RAPPORT DE RECHERCHE EUROPEENNE

Numero de la demande

EP 91 40 1866

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.5) |
|---|---|---|---|
| A | CHEMICAL ABSTRACTS, vol. 110, 1989, page 582, colonne 2, résumé no. 74763n, Columbus, Ohio, US; L.Y. TSUNG et al.: "Ultrasonic irradiation in a single-stage synthesis of chloroiodomethane from iodoalkanes and dichloromethane under aluminium mediation", & APPL. ORGANOMET. CHEM. 1988, 2(3), 239-43 * Résumé * --- | 1 | C 07 C 205/12 C 07 C 201/12 C 07 B 39/00 |
| A | US-A-4 140 719 (R.J. TULL et al.) * Revendications; colonnes 3-4 * --- | 23,24 | |
| A | FR-A-2 391 990 (BASF AG) * Revendication * ----- | 23,24 | |

DOMAINES TECHNIQUES RECHERCHES (Int. Cl.5)

C 07 C 205/00
C 07 C 201/00
C 07 B 39/00

Le présent rapport a été établi pour toutes les revendications

| Lieu de la recherche | Date d'achèvement de la recherche | Examinateur |
|---|---|---|
| LA HAYE | 03-09-1991 | BONNEVALLE E.I.H. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P0402)